# NEUE EUROPÄISCHE PATENTSCHRIFT

(11) **EP 0 491 757 B2**
(45) Veröffentlichungstag und Bekanntmachung des Hinweises auf die Entscheidung über den Einspruch: **09.01.2002**
(45) Hinweis auf die Patenterteilung: 07.01.1998
(21) Anmeldenummer: 90913337.3
(22) Anmeldetag: 08.09.1990
(51) Int. Cl.: A01N 1/02

(54) **VERFAHREN ZUR INAKTIVIERUNG VON VIREN IN BLUT UND BLUTPRODUKTEN**
PROCESS FOR INACTIVATING VIRUSES IN BLOOD AND BLOOD PRODUCTS
PROCEDE DE DESACTIVATION DE VIRUS DANS DU SANG ET DES PRODUITS DU SANG

(30) Priorität: 13.09.1989 DE 3930510
(43) Veröffentlichungstag der Anmeldung: 01.07.1992
(60) Teilanmeldung: 93120072.9
(73) Patentinhaber: BLUTSPENDEDIENST DER LANDESVERBÄNDE DES DEUTSCHEN ROTEN KREUZES NIEDERSACHSEN, OLDENBURG UND BREMEN G.G.m.b.H., D-31830 Springe (DE)
(72) Erfinder: MOHR, Harald, D-3000 Hannover 1 (DE); LAMBRECHT, Bernd, D-3257 Springe 4 (DE)
(74) Vertreter: Schupfner, Gerhard D., Dr. Dipl.-Chem.
(86) Internationale Anmeldenummer: DE9000691
(87) Internationale Veröffentlichungsnummer: WO9103933

(56) Entgegenhaltungen:
- EP-A- 0 196 515
- WO-A-90/07876
- WO-A-90/13296
- Proceedings of the Society for Experimental Biology and Medicine, Band 148, 1975, S. 291-293, Te-Wen Chang et al: "Photodynamic Inactivation of Herpesvirus Hominis by Methylene Blue (38524)"
- Concepts in Radiation Cell Biology, Kapitel 2, 1972, S. 57-89, C. W. Hiatt: "Methods for Photo-inactivation of Viruses"
- Photochemistry and Photobiology, vol. 29, 1979, W. Snipes et al: "Inactivation of lipid-containing viruses by hydrophobic photosensitizers and near-ultraviolet radiation", S. 785-790
- J. Gen. Virol., vol. 41, 1978, G. S. L. Yen et al: "Photosensitization of Herpes Simplex Virus Type 1 with Neutral Red", S. 273-281

## Beschreibung

Die Erfindung betrifft ein verfahren zur Inaktivierung von Viren in Blut und Blutprodukten, bei dem die zu behandelnden Lösungen bzw. Suspensionen mit Phenothiazinfarbstoffen versetzt und anschließend mit sichtbarem Licht im Bereich des Absorptionsmaximums des jeweiligen Farbstoffs bestrahlt und danach gegebenenfalls das Blut bzw. die Blutprodukte zur Entfernung der Farbstoffe über Adsorptionsmittel geleitet werden.

Es ist bekannt, daß photodynamische Substanzen in Verbindung mit sichtbarem Licht oder UV-Licht virusinaktivierend wirken können. Ursache hierfür ist die Affinität dieser Stoffe zu äußeren Virusstrukturen oder zur viralen Nukleinsäure. Beides trifft für Phenothiazinfarbstoffe zu. Sie reagieren mit dem Membranstrukturen umhüllter Viren und schädigen diese unter Lichteinfluß irreversibel, wodurch das Virus seine Infektiosität verliert (vgl. Snipes, W. et al., 1979, Photochem. and Photobiol. 29, 785-790).

Photodynamische Substanzen interagieren aber auch mit viraler RNA oder DNA, inbesondere mit den Guaninresten dieser Nukleinsäuren. Nach Bildung eines Farbstoff-Nukleinsäure-Komplexes wird dieser durch Lichtenergie angeregt, so daß es zur Denaturierung der Nukleinsäure und letztlich zu Strangbrüchen kommt Weiterhin bewirken Phenothiazinfarbstoffe die Umwandlung von molekularem Sauerstoff in Sauerstoffradikale, die hoch reaktiv sind und auf verschiedene Weise viruzid wirken können (vgl. Hiatt, C.W., 1972, in: Concepts in Radiation Cell Biology, pp. 57-89, Academic Press, New York; Oh Uigin et al., 1987, Nucl. Acid. Res. 15, 7411-7427).

Im Gegensatz zu anderen photodynamischen Farbstoffen zur Virusinaktivierung sind Phenothiazinfarbstoffe wie Methylenblau, Neutralrot und Toluidinblau deswegen von Bedeutung, weil sie schon in Verbindung mit sichtbarem Licht eine Reihe von Viren inaktivieren können, unter bestimmten Bedingungen auch solche, die keine Lipidhüllen besitzen, wie z. B. das Adenovirus.

Hinzu kommt, daß z.B. Methylenblau (MB) und Toluidinblau (TB) selbst therapeutische Anwendung finden, u.a. als Antidot bei Kohlenmonoxidvergiftungen und in der Langzeittherapie psychotischer Erkrankungen. Hierbei kommen ohne bedeutsame Nebenwirkungen Mengen von MB bzw. TB zur Anwendung (1 - 2 mg/kg Körpergewicht), die weit höher sind als diejenigen, die zur Virusinaktivierung notwendig sind. Die geringen Toxizitäten von MB und TB werden auch durch tierexperimentelle Daten belegt.

Seit dem Jahre 1955 wird in der Fachwelt jedoch davon ausgegangen, daß Farbstoffkonzentrationen, insbesondere bei Toluidinblau, unterhalb von 2,5 µM nur unzureichend virusinaktivierend wirken (vgl. F. Heihmets et al., lnactivation of Viruses in Plasma by Photosensitized Oxidation, Research Report, Walter Reed Army Institute of Research, Walter Reed Army Medical Center, Washington D.C., 1955).

Bei den bisher beschriebenen Untersuchungen zur Virusinaktivierung mit Phenothiazinfarbstoffen liegen die Farbstoffkonzentrationen zwischen 10 µM und 100 µM (Chang and Weinstein, 1975, Photodynamic Inactivation of Herpesvirus Hominis by Methylene Blue (38524), Proceedings of the Society for Experimental Biology and Medicine, 148:291-293; Yen and Simon, 1978, Photosensitization of Herpes Simplex Virus Type 1 with Neutral Red, J. gen. Virol., 41:273-281). Bei diesen Konzentrationen besteht aber der Nachteil, daß es nicht nur zur Virusinaktivierung, sondern auch zur Inaktitivierung von Plasmaproteinen wie z.B. der Gerinnungsfaktoren kommt. Dies ist einer der Gründe, weshalb Phenothiazinfarbstoffe bislang keine Rolle bei der Virusinaktivierung in Blut und Blutprodukten erlangt haben.

Die Aufgabe der vorliegenden Erfindung besteht nun darin, ein Verfahren zur Virusinaktivierung zu entwickeln, bei dem Viren verschiedener Art durch Einsatz von Phenothiazinfarbstoffen aber ohne funktionelle Beeinträchtigungen der Plasmaproteine abgetötet werden. Des weiteren besteht die Aufgabe der Erfindung darin, das Verfahren so einfach zu gestalten, daß Blut bzw. Blutprodukte unmittelbar in handelsüblichen Blutbeuteln behandelt werden können.

Erfindungsgemäß wird die gestellte Aufgabe dadurch gelöst, daß die Phenothiazinfarbstoffe in einer Konzentration von 0,1 bis 2 µM eingesetzt werden und die Bestrahlung unmittelbar in transparenten Behältnissen wie Blutbeuteln erfolgt, die zur Blutentnahme und Aufbewahrung dienen, wobei die Viren keine nicht umhüllten Viren sind, oder die Aufgabe gemäß Anspruch 7 gelöst wird.

Vorteilhafte Ausgestaltungen der Erfindung sind in den Unteransprüchen beschrieben.

Die Bestrahlung erfolgt mit Tageslicht ausreichender Stärke oder mit monochromatischem Licht vorzugsweise einer Kaltlichtquelle, die eine Wellenlänge im Bereich des Absorptionsmaximus des jeweiligen Farbstoffes besitzt. Ferner sollten folgende Bedingungen bei der Virusinaktivierung im Blutplasma bzw. in Plasmaproteinlösungen eingehalten werden. Die Arbeitstemperatur sollte im Bereich von 0 bis 37 °C, möglichst aber im Bereich von 4 bis 20 °C liegen. Die Inaktivierungsdauer beträgt insbesondere 5 Minuten bis 5 Stunden, vorzugsweise 10 Minuten bis 3 Stunden, und der pH-Wert soll zwischen pH 5 und pH 9 liegen, vorzugsweise zwischen pH 6 und pH 8.

Ein wesentlicher Vorteil des erfindungsmäßigen Verfahrens liegt in seiner Einfachheit F. Heinmets et al. ( wie vorn angegeben) beschreiben eine hochaufwendige Apparatur, durch die z.B. Blutplasma geleitet werden muß. Dabei treten Wartungs- und vor allem auch Kapazitätsprobleme auf. Überraschenderweise wurde nun festgestellt, daß man mit wesentlich geringeren Mengen an Farbstoff auskommen kann und zudem eine aufwendige technische Einrichtung zur Photoinaktivierung nicht benötigt wird.

Unerwartet war auch die Feststellung, daß ein nicht umhülltes Virus wie Adeno, das sich unter physiologischen Umständen in Plasma nicht inaktivieren ließ, durch einen Frieren/Tauen-Schritt photosensitiviert und somit inaktiviert werden konnte. Dabei konnte die Inaktivierung, unabhängig von der Reihenfolge der Arbeitsschritte Frieren/Tauen und Zugabe des Farbstoffes, festgestellt werden. Unter Frieren wird ein Tiefgefriervorgang mit verflüssigten Gasen als Kältemittel bei Temperaturen von etwa - 20 °C bis etwa 80 K verstanden. In der Regel wird bei Temperaturen unter - 30 °C tiefgefroren.

Man kann die Virusinaktivierung unmittelbar in Blut- bzw. Plasmabeuteln durchführen, obwohl diese nur begrenzt lichtdurchlässig sind. Es muß lediglich der Farbstoff zugefügt werden. Dann wird der Beutel samt Inhalt belichtet, und daraufhin kann das jeweilige Produkt weiterprozessiert werden.

Das Verfahren ist also ohne größeren technischen Aufwand durchzuführen und an Blutbanken hervorragend in den Arbeitsablauf bei der Prozessierung von einzelnen Blutspenden integrierbar. Die geringe Menge des eingesetzten Farbstoffs kann in der behandelten Flüssigkeit verbleiben oder wird durch Adsorptionsmittel entfernt.

Als Blut bzw. Blutprodukte kommen unter anderem in Frage:
- Vollblut
- Erythrozytenkonzentrate
- Thrombozytenkonzentrate
- Plasma
- Serum
- Kryopräzipitat
- Konzentrate von Gerinnungsfaktoren
- Inhibitoren
- Fibronectin
- Albumin.

Zur Anwendung des erfindungsgemäßen Verfahrens sind Phenothiazine der folgenden Strukturformel geeignet.

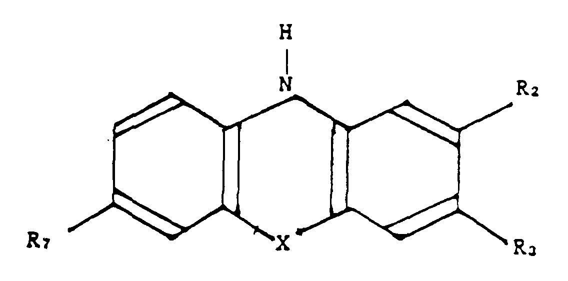

| | X | R₂ | R₃ | R₇ |
|---|---|---|---|---|
| Neutralrot | N | CH₃ | NH₂ | N(CH₃)₂ |
| Toluidinblau | S | CH₃ | NH₂ | N(CH₃)₂ |
| Methylenblau | S | H | N(CH₃)₂ | N(CH₃)₂ |
| Phenothiazin | S | H | H | H |

### Beispiel 1

Mit Methylenblau (MB) wird nachfolgend die Konzentrationsabhängigkeit der Photoinaktivierung beim Vesikular-Stomatitis-Virus (VSV) im Humanplasma aufgezeigt.

Ca. 5 x 10⁷ Plaque Forming Units (PFU) pro ml VSV wurden in Humanplasma suspendiert und mit verschiedenen Konzentrationen an MB versetzt. Kontrollproben enthielten keinen Farbstoff. Das Probevolumen betrug 0,5 ml. Eine Kontrollprobe und ein Teil der MB-haltigen Proben wurden für 4 h bei Raumtemperatur mit sichtbarem Licht bestrahlt; die anderen wurden genauso lange im Dunkeln gelagert. Als Lichtquelle diente ein Diaprojektor, der mit einer 150 W Halogenbirne (Osram Xenophot) ausgestattet war. Der Abstand zwischen dem Objektiv des Diaprojektors, also der Lichtauslaßöffnung und den Proben betrug bei diesen und allen weiteren Versuchen 30 cm. (Mit Ausnahme der Virusinaktivierung in Blutbeuteln).

Nach Ablauf der Bestrahlung wurde in allen Proben über einen Plaque-Assay der Virustiter bestimmt. Als Indikatorzellen dienten BHK-Zellen. Die Versuchsergebnisse sind in der Tabelle 1 aufgelistet.

**Tab. 1:**

| Inaktivierung von VSV in Humanplasma mit und ohne Belichtung. Belichtungsdauer: 4 h | | | |
|---|---|---|---|
| Proben | MB-Konzentration (µM) | Licht | Virusinaktivierungsfaktor |
| Kontr. 1 | 0 | + | 4,8 |
| Kontr. 2 | 0 | - | 1 |
| 1 | 0,01 | + | 11,8 |
| 2 | 0,1 | + | 28,5 |
| 3 | 0,5 | + | > 10⁶ |
| 4 | 1 | + | > 10⁶ |
| 5 | 10 | + | > 10⁶ |
| 6 | 50 | + | > 10⁶ |
| 7 | 100 | + | > 10⁶ |
| 8 | 1 | - | 1 |
| 9 | 10 | - | 5 |
| 10 | 50 | - | 11,8 |
| 11 | 100 | - | 95 |

Die Ergebnisse aus Tab. 1 zeigen, daß ab einer Konzentration von MB um 0,5 µM der infektiöse Titer von VSV um mehr als 6 Zehnerpotenzen vermindert wurde. Deutlich höhere Konzentrationen des Farbstoffes, ab etwa 50 µM, führten bereits ohne Belichtung zu einer signifikaten Verringerung des VSV-Titers.

### Beispiel 2

Durch den folgenden Versuch wurde die Virusinaktivierung bei niedrigen Farbstoffkonzentrationen bestätigt.

VSV wurde in Gegenwart von Plasma und verschiedenen Mengen Methylenblau in Aliquots von 500 µl Volumen über Nacht im Kühlraum aus einem Abstand von 30 cm mit dem Diaprojektor bestrahlt. Die Proben A - F wurden belichtet, Probe G blieb unbelichtet.

Die Ergebnisse dieses Versuchs sind in Tab. 2 dargestellt. Sie zeigen, daß unter den oben genannten Bedingungen das eingesetzte VSV um mehr als 4 Zehnerpotenzen inaktiviert wurde. Dazu waren 0,5 µM Methylenblau notwendig.

Wahrscheinlich hat allein durch die Inkubation über Nacht bei 4 Grad Celsius der Titer des VSV um 1 - 2 Zehnerpotenzen abgenommen, was den relativ niedrigen Ausgangstiter erklären würde. Dies wurde hierbei allerdings nicht mitgetestet.

Der Vergleich von A (belichtet) und G (dunkel) zeigt, daß Licht allein offenbar keinen großen Einfluß auf die Infektiosität des Virus ausübt.

**Tab. 2:**

| Virusinaktivierung bei niedrigen Farbstoffkonzentrationen | | | |
|---|---|---|---|
| Probe | MB-Endkonz. µM | Titer/200 µl | Inaktivierungsfaktor |
| A | 0 | 2 x 10⁴ | 2,2 |
| B | 0,01 | 2,4 x 10⁴ | 1,8 |
| C | 0,05 | 2 x 10⁴ | 2,2 |
| D | 0,25 | 3 x 10² | 147 |
| E | 0,5 | < 1 | ≥ 4,4 x 10⁴ |
| F | 1,0 | < 1 | > 4,4 x 10⁴ |
| G | 0 | 4,4 x 10⁴ | 1 |

### Beispiel 3

Die Photoinaktivierung bei Viren in Gegenwart von Phenothiazinfarbstoffen ist jedoch abhängig von der Dauer der Belichtung. Um zu untersuchen, welche Belichtungszeiten zur Photoinaktivierung von VSV ausreichen, wurden 10⁶ Plaque Forming Units (PFU) pro ml in Plasma suspendiert und für verschiedene Zeiten bei 22 °C wie beschrieben belichtet. Die erhaltenen Ergebnisse zeigt die Tabelle 3. Man erkennt, daß unter den gegebenen Versuchsbedingungen eine Stunde Belichtungszeit ausreichte, um den infektiösen VSV-Titer um mehr als 6 Zehnerpotenzen zu vermindern.

**Tab. 3:**

| Kinetik der MB-vermittelten Photoinaktivierung von VSV | | |
|---|---|---|
| Probe | Belichtungszeit (min) | Inaktivierungsfaktor |
| Kontrolle | 0 | 1 |
| 1 | 5 | 50 |
| 2 | 30 | 1666 |
| 3 | 60 | > 10⁵ |

### Beispiel 4

Ein ähnlicher Versuch wurde anstatt mit MB in Gegenwart von 1 µM eines anderen Phenothiazinfarbstoffes, TB, durchgeführt. Die in der Tabelle 4 dargestellten Ergebnisse zeigen, daß auch mit Hilfe von TB VSV wirkungsvoll inaktiviert werden kann.

**Tab. 4:**

| Kinetik der TB-vermittelten Photoinaktivierung von HSV | | |
|---|---|---|
| Probe | Belichtungszeit (min) | Inaktivierungsfaktor |
| Kontrolle | 0 | 1 |
| 1 | 10 | 20 |
| 2 | 60 | > 4 x 10³ |

Die inaktivierende Wirkung der Phenothiazinfarbstoffe wurde auch beim Herpex-Simplex-Virus (HSV) sowie beim Human Immunodeficiency Virus Typ 1 (HIV-1) nachgewiesen.

### Beispiel 5

In Gegenwart von Methylenblau (1 µM) wird auch HSV inaktiviert. Tab. 5 zeigt die Kinetik der MB-vermittelten Photoinaktivierung von HSV.

**Tab. 5:**

| Kinetik der MB-vermittelten Photoinaktivierung von HSV | | |
|---|---|---|
| Probe | Belichtungszeit (min) | Inaktivierungsfaktor |
| Kontrolle | 0 | 1 |
| 1. | 20 | 35 |
| 2 | 60 | 1500 |
| 3 | 180 | > 3 x 10⁴ |

### Beispiel 6

Ein ähnlicher Versuch wurde mit dem AIDS-Erreger HIV-1 durchgeführt. Der Virustiter betrug 6 x 10² PFU/ml. Als Indikatorzellen dienten MT4-Zellen. Die Tabelle 6 zeigt, daß HIV-1 besonders empfindlich gegenüber der Photoinaktivierung zu sein scheint: bereits innerhalb von 10 min wurde der Virustiter um mehr als das 600-fache reduziert.

**Tab. 6:**

| Kinetik der MB-vermittelten Photoinaktivierung von HIV-1 | | |
|---|---|---|
| Probe | Belichtungszeit (min) | Inaktivierungsfaktor |
| Kontrolle | 0 | 1 |
| 1 | 10 | > 600 |
| 2 | 60 | > 600 |
| 3 | 120 | > 600 |

### Beispiel 7

Bei dem Versuch, nicht umhüllte Viren unter den üblichen physiologischen Bedingungen in Gegenwart von 80 % Plasma zu inaktivieren, konnte kein Erfolg erzielt werden. Adenovirus wurde, als Modell für ein nicht umhülltes Virus, längere Zeiten (4 °C, dunkel) in Gegenwart des Farbstoffes Methylenblau (MB), 1 µM, vorinkubiert. Danach erfolgte eine 30minütige Bestrahlung mit Halogenstrahlern (150 000 Lux). Dabei blieb die Infektiosität des Adenovirus unverändert.

**Tab. 7:**

| Einfluß der Vorinkubationsdauer auf die Photosensitivierung von Adenovirus. | | | |
|---|---|---|---|
| Probe | Dauer der Vorinkubation | Farbstoff | Titer (log10) |
| Kontrolle | 0 h | -- | 6,0 |
| 1 | 0 h | MB | 6,0 |
| 2 | 1 h | MB | 5,5 |
| 3 | 4 h | MB | 6,0 |
| 4 | 24 h | MB | 6,0 |

Der Titer wurde als TCID50 (Berechnungsmethode "Tissue Culture Infectious Dosis" nach Spearman und Kaerber) bestimmt. Das Virus wurde auf FL-Zellen (definierte Zelllinie zur Virustitration geeignet) titriert.

Bei der Verwendung von Toluidinblau unter Beibehaltung der experimentellen Bedingungen konnte ebenfalls keine Reduktion des Virustiters festgestellt werden.

Um eine Inaktivierung von Adenovirus zu erzielen, wurde ein Frieren/Tauen-Schritt (F/T) unter Tiefgefrieren auf30 °C in den Versuchsablauf eingefügt. Dabei spielte die Reihenfolge des F/T und die Zugabe des Farbstoffes (1 µM MB) nur eine untergeordnete Rolle. Die Bestrahlung der Proben erfolgte gleichfalls mit Hilfe der Halogenstrahler. Gemessen wurden 120 000 Lux.

**Tab. 8:**

| Photosensitivierung von Adenovirus aufgrund eines eingefügten F/T-Schrittes. | | |
|---|---|---|
| Probe | Probenvorbereitung | Titer (log10) |
| Kontrolle | | 7,5 |
| A | F/T | 7,0 |
| B | F/T + 60 min Bestrahlung (Bestr.) | 7,5 |
| C | F/T + MB + 60 min Vorinkubat. + 60 min Bestr. | 2,5 |
| D | MB + F/T | 7,5 |
| E | MB + F/T + 10 min Bestr. | 5,0 |
| F | MB + F/T + 30 min Bestr. | 5,0 |
| G | MB + F/T + 60 min Bestr. | 4,0 |

Die Durchführung der Virustitration erfolgte wie in Tab. 7 beschrieben.

### Beispiel 8

Das besondere Problem bei der Anwendung hoher Farbstoffkonzentrationen liegt in der unmittelbaren Wirkung dieser Stoffe auf Plasmaproteine. In einem weiteren Versuch wurden daher unterschiedliche Farbstoffkonzentrationen im Hinblick auf ihre Wirkung gegenüber der Aktivität von Gerinnungsfaktoren untersucht.

Humanes Plasma (2 ml Aliquots) wurde mit verschiedenen Mengen von MB versetzt. Direkt danach wurden die Aktivitäten der Gerinnungsfaktoren V, VIII und IX gemessen. Wie aus der Tabelle 9 ersichtlich ist, werden sie in allen drei Fällen dosisabhängig inhibiert, die der Faktoren VIII und V ab etwa 10 µM und die des Faktors IX bereits ab 2,5 µM. Demnach wirkt bei höheren Konzentrationen MB direkt auf die Proteine, ohne daß Licht einwirken muß.

**Tab. 9:**

| Einfluß von MB auf die Aktivität von Gerinnungsfaktoren | | | |
|---|---|---|---|
| Methylenblau (µM/l) | Faktor V E/ml | Faktor VIII E/ml | Faktor IX E/ml |
| 0 | 0,80 | 0,38 | 2,0 |
| 1 | 0,76 | 0, 41 | 1,9 |
| 2,5 | 0,78 | 0,41 | 1,6 |
| 5 | 0,74 | 0,38 | 1,45 |
| 10 | 0,54 | 0,35 | 1,20 |
| 20 | 0,44 | 0,28 | 1,10 |

### Beispiel 9

Nun hat aber nicht nur die eingesetzte Farbstoffkonzentration, sondern auch die Belichtungszeit einen Einfluß auf die Aktivität von Gerinnungsfaktoren. Diese Zeitabhängigkeit wurde, bei verschiedenen Methylenblaukonzentrationen ermittelt.

Humanes Plasma (2 ml-Aliquots) wurden mit verschiedenen Mengen an MB versetzt und für 1 bis 4 h belichtet (wie im Beispiel 1 beschrieben). Kontrollproben wurden nicht photobehandelt. Wie die Tabelle 10 zeigt, werden die Aktivitäten der drei Gerinnungsfaktoren V, VIII und IX zeit- und dosisabhängig inhibiert. Besonders bei den Faktoren VIII und IX zeigt sich, daß höhere MB-Konzentrationen und Licht-Expositionszeiten ab 2 h eine anscheinende Erhöhung der thrombolytischen Aktivitäten bewirken.

**Tab. 10:**

| Einfluß von Licht und MB auf die Aktivität von Gerinnungsfaktoren: Zeit- und Dosisabhängigkeit | | | | |
|---|---|---|---|---|
| Belichtungszeit | Konzentration Methylenblau µM/l | Faktor V E/ml | Faktor VIII E/mi | Faktor IX E/ml |
| 0 h | 0 | 0,86 | 0,33 | 1,20 |
| | 1 | 0,86 | 0,45 | 1,20 |
| | 2,5 | 0,82 | 0,33 | 0,46 |
| | 10 | 0,72 | 0,30 | 0,44 |
| | | | | |
| 1 h | 0 | 0,84 | 0,40 | 0,76 |
| | 1 | 0,72 | 0,24 | 0,92 |
| | 2,5 | 0,68 | 0,24 | 0,32 |
| | 10 | 0,47 | 0,16 | 0,68 |
| | | | | |
| 2 h | 0 | 0,82 | 0,44 | 0,10 |
| | 1 | 0,64 | 0,23 | 0,90 |
| | 2,5 | 0,68 | 0,22 | 0,72 |
| | 10 | 0,60 | 0,15 | 0,74 |
| | | | | |
| 4 h | 0 | 0,76 | 0,38 | 0,98 |
| | 1 | 0,56 | 0,16 | 0,94 |
| | 2,5 | 0,49 | 0,29 | 0.82 |
| | 10 | 0,42 | 0,27 | 0,64 |

### Beispiel 10

Gemäß einer bevorzugten Ausführungsform der vorliegenden Erfindung kann die Photoinaktivierung von Viren unmittelbar im Plasmabeutel vorgenommen werden. Dem Blut oder den Blutprodukten wird lediglich der Farbstoff in erforderlicher Menge zugesetzt und der Beutel dann mit Licht bestrahlt. Auf diese einfache Weise lassen sich Blutprodukte von einzelnen Spendern jederzeit behandeln.

In einem Versuch wurden drei Proben eines humanen Frischplasmas aufgetaut und in den Plasmabeuteln mit jeweils 1,5 x 10⁶ PFU VSV versetzt. Zu zwei Proben wurde MB in Konzentrationen von 1 bzw. 10 µM zugefügt. Aus dem MB-freien Plasma wurde eine Probe entnommen und als Positiv-Kontrolle im Dunkeln bei 4 °C aufbewahrt. Dann wurden die drei Beutel zwischen zwei Plexiglasplatten eingespannt, um eine möglichst gleichmäßige Schichtdicke von ca. 2,5 cm zu gewährleisten. Sie wurden daraufhin aus einer Entfernung von ca. 90 cm mit Hilfe eines Diaprojektors bestrahlt. Nach 4 h wurden zur Bestimmung des Virustiters Proben entnommen und dieser mittels Plaque-Assay auf FL-Zellen gemessen. Die Ergebnisse in Tab. 11 zeigen, daß bereits 1 µM MB ausreicht, um im Plasmabeutel durch vierstündige Belichtung den infektiösen Titer von VSV um mehr als drei Zehnerpotenzen zu reduzieren. Auch bei Abwesenheit des Farbstoffes führte die Belichtung zu einer Reduktion des Virustiters, allerdings nur um etwa 50 %.

**Tab. 11:**

| Photoinaktivierung von VSV im Plasmabeutel | | | | |
|---|---|---|---|---|
| Probe | Bestrahlungsdauer (h) | MB-Konzentration (µM) | Beutel-Gewicht (g) | VSV-Titer (PFU/ml) |
| Kontrolle | 0 | 0 | 323 | 5 x 10³ |
| 1 | 4 | 0 | 323 | 2,5 x 10³ |
| 2 | 4 | 1 | 289 | 0 |
| 3 | 4 | 10 | 257 | 0 |

Die zur Virusinaktivierung eingesetzten Phenothiazinfarbstoffe können insbesondere bei den hier angewendeten Konzentrationen von bis zu 1 µM im Blut oder den Blutprodukten verbleiben, ohne daß es zu Nebenwirkungen kommt. Sie lassen sich aber auch nachträglich über Dialyse, Gelfiltration oder Adsorption wieder entfernen.

Von den genannten Methoden sind vor allem die adsorptiven von Interesse, weil sie den geringsten zeitlichen und technischen Aufwand machen und die betreffenden Plasmaproteinlösungen nicht verdünnt werden.

### Beispiel 11

### Säulenchromatographische Entfernung von MB aus Plasmaproteinlösungen

Bei diesem Versuch sollte herausgefunden werden, ob die adsorptive Entfernung des Photooxidans auch auf chromatographischem Wege erfolgen kann. Hintergrund ist die Idee, daß man die Virusinaktivierung mittels Farbstoff in Kombination mit Licht in einem Behältnis durchführt, beispielsweise einem Blutbeutel und dann die Plasmaproteinlösung über eine kleine dazwischengeschaltete Trennsäule, die das Adsorptionsmittel enthält, in ein weiteres Behältnis, z.B. einen zweiten Blutbeutel, überführt. Ware jetzt die Einheit erster Beutel -Adsorptionssäule - zweiter Beutel vorgefertigt, verfügte man also über ein geschlossenes System, und man könnte auf einfache Weise unter geringstem Kontaminationsrisiko virusinaktivierte Plasmaproteinpräparate, auch von einzelnen Donoren stammend, herstellen.

Hierzu wurden 250 ml 5%iger Albuminlösung mit verschiedenen Geschwindigkeiten durch eine Trennsäule geleitet, die 5 ml Kieselgel (Porenweite 40 A) enthielt. 10 ml-Fraktionen wurden aufgefangen und ihre Extinktionswerte bei 660 nm gemessen.

Wie aus der Tabelle 12 hervorgeht, ließ sich das Gesamtvolumen der Albuminlösung bei Durchflußgeschwindigkeiten von 5 bzw. 7,5 ml/min durch die Säule leiten, ohne daß restliches MB in den Durchlauffraktionen nachzuweisen war. Der Zeitaufwand, um aus 250 ml Lösung den Farbstoff zu entfernen, liegt demzufolge bei höchstens 30 bis 35 Minuten.

Das Ergebnis des Versuches zeigt, daß die chromatographische Abtrennung des Photooxidans keine Probleme bereitet, zum anderen wird belegt, daß die oben skizzierte Herstellung virusinaktivierter Plasmaproteinpräparate einzelner Donoren möglich ist.

**Tab. 12:**

| Chromatographische Abtrennung von MB aus einer 5%igen Albuminlösung (MB-Konzentration 1 µM). | | |
|---|---|---|
| Ausgangsmaterial + MB | Durchlaufgeschwingigkeit (ml/min) | |
| | 5 | 7.5 |
| Extinktion (660 nm): 0,067 | Extinktion (660 nm) | |
| Fraktion-Nr.: 1 | 0.002 | 0,001 |
| 3 | 0.000 | 0,001 |
| 5 | 0.000 | 0.002 |
| 7 | 0.002 | 0.003 |
| 9 | 0.001 | 0.001 |
| 11 | 0.000 | 0.001 |
| 13 | 0.000 | 0.001 |
| 14 | 0,002 | 0.001 |
| | | |

## Patentansprüche

1. Verfahren zur Inaktivierung von Viren in Blut und Blutprodukten, bei dem die zu behandelnden Lösungen bzw. Suspensionen mit Phenothiazinfarbstoffen versetzt und anschließend mit Licht bestrahlt werden, **dadurch gekennzeichnet, daß** die Phenothiazinfarbstoffe in einer Konzentration von 0,1 bis 2 µM eingesetzt werden und die Bestrahlung unmittelbar in transparenten Behältnissen wie Blutbeuteln erfolgt, die zur Blutentnahme und Aufbewahrung dienen, wobei die Viren keine nicht umhüllten Viren sind.

2. Verfahren nach Anspruch 1, **dadurch gekennzeichnet, daß** als Phenothiazinfarbstoff Toluidinblau oder Methylenblau verwendet wird.

3. Verfahren nach einem der Ansprüche 1 oder 2, **dadurch gekennzeichnet, daß** die zu behandelnden Lösungen bzw. Suspensionen zunächst tiefgefroren und dann vor der Bestrahlung wieder aufgetaut werden.

4. Verfahren nach Anspruch 3, **dadurch gekennzeichnet, daß** der Farbstoff vor dem Gefriervorgang zugesetzt wird.

5. Verfahren nach Anspruch 3, **dadurch gekennzeichnet, daß** der Farbstoff nach dem Auftauen und vor der Bestrahlung zugegeben wird.

6. Verfahren nach einem der Ansprüche 1 bis 5, **dadurch gekennzeichnet, daß** dieses über zwei zur Blutentnahme geeignete Behältnisse wie Blütbeutel mit einer dazwischengeschalteten Trennsäule, die Adsorptionsmittel für die Phenothiazinfarbstoffe enthält, durchgeführt wird.

7. Verfahren zur Inaktivierung von Viren in Blut und Blutprodukten, bei dem. die zu behandelnden Lösungen bzw. Suspensionen mit Phenothiazinfarbstoffen versetzt und anschließend mit Licht bestrahlt werden, **dadurch gekennzeichnet, daß**
- die Phenothiazinfarbstoffe in einer Konzentration von 0,1 bis 2 µM eingesetzt werden,
- die Bestrahlung unmittelbar in transparenten Behältnissen wie Blutbeuteln erfolgt, die zur Blutentnahme und Aufbewahrung dienen und
- die zu behandelnden Lösungen bzw. Suspensionen zunächst tiefgefroren und dann vor der Bestrahlung wieder aufgetaut werden.

## Claims

1. A process for inactivating viruses in blood and blood products, comprising adding phenothiazine dyes to the solutions or suspensions to be treated and subsequently irradiating said phenothiazine dye-containing solutions or suspensions with light, **characterised in that** the phenothiazine dyes are used at a concentration of from 0.1 to 2 µM and irradiation is effected directly in transparent containers, such as blood bags, used for collecting and storing blood, wherein the viruses are not nonenveloped viruses.

2. The process as claimed in claim 1, **characterised in that** toluidine blue or methylene blue is used as said phenothiazine dye.

3. The process as claimed in any one of the claims 1 or 2, **characterised in that** the solutions or suspensions to be treated are initially subjected to deep-freezing and then are thawed prior to irradiation.

4. The process as claimed in claim 3, **characterised in that** the dye is added prior to the freezing step.

5. The process as claimed in claim 3, **characterised in that** the dye is added after thawing and prior to irradiation.

6. The process as claimed in any one of the claims 1 to 5, **characterised in that** said process is carried out using two containers suitable for collecting blood, such as blood bags, with a separating column interposed between them and containing an adsorbing agent for the phenothiazine dyes.

7. A process for inactivating viruses in blood and blood products, comprising adding phenothiazine dyes to the solutions or suspensions to be treated and subsequently irradiating said phenothiazine dye-containing solutions or suspensions with light, **characterised in that**
- the phenothiazine dyes are used at a concentration of from 0.1 to 2 µM,
- irradiation is effected directly in transparent containers, such as blood bags, used for collecting and storing blood and
- the solutions or suspensions to be treated are initially subjected to deep-freezing and then are thawed prior to irradiation.

## Revendications

1. Procédé d'inactivation de virus dans le sang et les produits du sang, dans lequel les solutions ou les suspensions à traiter sont mélangées avec des colorants à la phénothiazine et, ensuite, irradiées par la lumière, **caractérisé en ce que** les colorants à la phénothiazine sont utilisés à une concentration de 0,1 à 2 µM, et **en ce que** l'irradiation se produit directement dans des récipients transparents, comme les poches à sang qui servent au prélèvement et à la conservation du sang, les virus n'étant pas des virus sans enveloppe.

2. Procédé suivant la revendication 1, **caractérisé en ce que** le bleu toluidine ou le bleu de méthylène sont utilisés comme colorants à la phénothiazine.

3. Procédé suivant l'une quelconque des revendications 1 ou 2, **caractérisé en ce que** les solutions ou les suspensions à traiter sont d'abord congelées, puis décongelées avant l'irradiation.

4. Procédé suivant la revendication 3, **caractérisé en ce que** le colorant est ajouté avant le processus de congélation.

5. Procédé suivant la revendication 3, **caractérisé en ce que** le colorant est ajouté après la décongélation et avant l'irradiation.

6. Procédé suivant l'une quelconque des revendications 1 à 5, **caractérisé en ce qu'**il est réalisé au moyen de deux récipients convenant au prélèvement du sang, comme des poches à sang, avec une colonne à fractionner intercalée, qui contient un moyen d'adsorber le colorant à la phénothiazine.

7. Procédé d'inactivation de virus dans le sang et dans les produits du sang, dans lequel les solutions ou les suspensions à traiter sont mélangées avec des colorants à la phénothiazine et ensuite irradiées par la lumière, **caractérisé en ce que** :
- les colorants à la phénothiazine sont utilisés à une concentration de 0,1 à 2 µM,
- l'irradiation s'effectue directement dans des récipients transparents comme des poches à sang qui servent au prélèvement et à la conservation du sang, et
- les solutions ou les suspensions à traiter sont d'abord congelées et puis à nouveau décongelées avant l'irradiation.
